# EUROPEAN PATENT APPLICATION

(11) **EP 4 818 162 A1**
(43) Date of publication of application: **07.10.2026**
(21) Application number: 25184447.8
(22) Date of filing: 23.06.2025
(51) Int. Cl.: C07D 251/60, C07D 251/62

(54) **PROCESS FOR THE SYNTHESIS OF MELAMINE WITH AN OPTIMIZED DISSOLUTION AND AMMONOLYSIS STEP**

(30) Priority: 03.04.2025 IT 202500007137
(71) Applicant: EUROTECNICA CONTRACTORS AND ENGINEERS S.p.A., 20159 Milano (IT)
(72) Inventor: DE AMICIS, Alberto, 20097 San Donato Milanese (MI) (IT); DI RUOCCO, Giuseppe, 23900 Lecco (LC) (IT); SANTUCCI, Roberto, I - 21050 Gorla Maggiore (VA) (IT); SERRAFERO, Carlo Alberto, 20098 San Giuliano Milanese (MI) (IT); PETRILLO, Francesca, 20161 Milano (IT)
(74) Representative: Bird & Bird Società tra Avvocati S.r.l.

(57) **Abstract**

The present invention relates to a process for the synthesis of melamine by the pyrolysis of urea, with an optimized dissolution and ammonolysis step. In particular a stream of a post-reactor section is fed to a dissolution step in aqueous ammonia solution, wherein the quantity of ammonia in the aqueous ammonia solution varies within the range of 15 to 28% by weight with respect to the total weight of the aqueous ammonia solution, in a dissolver (MD) at a temperature within the range of 160 to 180°C and at a pressure higher than 25 barg,

## Description

The present invention relates to a process for the synthesis of melamine with an optimized dissolution and ammonolysis step and the relative apparatus.

More specifically, the invention relates to a process which involves the synthesis of melamine at high pressure and the collection and purification in aqueous solution of the melamine produced in the pyrolysis reactor, and also its separation by crystallization.

It is known that the transformation of molten urea into molten melamine is described by the following overall reaction:

6 CO(NH₂)₂ → (CN)₃(NH₂)₃ + 6 NH₃ + 3CO₂ urea melamine off-gas

according to which for every kilogram of molten melamine 1.86 kilograms of NH₃ and CO₂ are formed, globally called off-gases.

One of the most widespread industrial processes based on the high-pressure pyrolysis of urea is that described in US patent 3,161,638 in the name of Allied. In this process, all the biphasic effluent from the melamine synthesis reactor is cooled and collected in an aqueous ammonia medium.

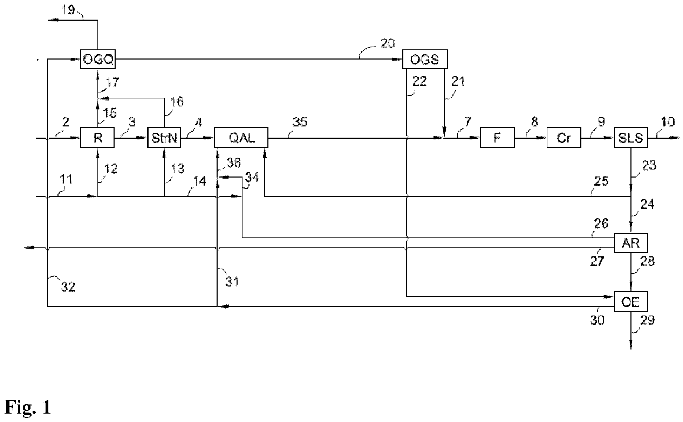

In a subsequent patent EP2385043B1, a low-energy consumption process for the production of high-purity melamine by the pyrolysis of urea is described and claimed, represented in one of its possible embodiments in figure 1.

The block diagram in figure 1 shows the main sections of a melamine production plant and the main streams of material according to the process described in EP2385043B1.

A stream 2 of liquid urea at a temperature higher than the melting point (equal to approximately 133°C) and a stream 12 of anhydrous gaseous NH₃ are sent to a reaction section R. The section R comprises a reactor equipped with a suitable heating system that keeps the reagent system at a temperature of approximately 360-420°C; the pressure is kept at a value higher than 70 barg.

Inside the reactor, or in one or more separators downstream (not shown in figure 1), the two-phase liquid/gas effluent produced by the urea pyrolysis reaction is separated into a liquid stream 3 of raw melamine comprising unreacted urea, NH₃, CO₂ and impurities such as OATs and polycondensates, and a first anhydrous off-gas stream 15 comprising saturated NH₃, CO₂ and melamine vapour.

The liquid stream 3 of raw melamine and the stream of anhydrous off-gases 15 are subjected to two different treatments for the recovery of the melamine contained in them.

The liquid stream 3 of raw melamine is sent to a stripping section StrN, which preferably operates under the same temperature and pressure conditions as section R, where it is placed in contact with a stream 13 of anhydrous gaseous NH₃. The mass ratio between said stream 13 and said stream 3 ranges from 0.06 to 0.60, and is preferably equal to 0.20.

The stream of anhydrous gaseous NH₃ flows in close contact with the liquid raw melamine stream and extracts the CO₂ dissolved therein up to a residual concentration of approximately 200 ppmw.

A liquid melamine stream (stream 4) exits from the section StrN, which is not only practically free of CO₂, but also partially purified as it is practically free of urea and contains small quantities of OATs and polycondensates. A second stream of anhydrous off-gases (stream 16) also exits from the section StrN, mainly comprising NH₃, a little CO₂ and a certain quantity of melamine vapour.

The anhydrous off-gas streams from the sections R and StrN, together or separately, preferably together and in this case the second anhydrous off-gas stream 16 is combined with the first anhydrous off-gas stream 15 forming a single stream 17, are subjected to a melamine recovery treatment by washing with water in a single washing section OGQ or in separate washing sections (not shown in figure 1).

As already indicated, the liquid stream 4 of melamine substantially free of CO₂ also exits from the stripping section with NH₃ StrN. It is sent to a quench-ammonolysis section QAL, where the quench (dissolution of the raw melamine in water) and ammonolysis (elimination of the polycondensates) treatments take place (figure 1).

The section QAL can consist of one or more stationary apparatuses, and preferably a single apparatus. The stream 4 enters the bottom of this apparatus, kept under vigorous stirring, and is put in close contact with an aqueous ammonia solution (stream 36), in which it is totally dissolved at a temperature of 160-180 °C, preferably 170-172 °C. The stirring and contact between streams 4 and 36 can be obtained by means of distributors, static mixers, fillings, internal stirrers, external circulation pumps or any other system typically used in the chemical industry for promoting the complete mixing of multiple fluid streams.

The aqueous stream 36 is formed by the joining of an aqueous stream (stream 31) coming from the successive steps of the melamine process and an NH₃ stream (stream 34), formed in turn by the joining of the NH₃ stream 26, also coming from the successive steps of the melamine process and the NH₃ replenishment stream 11; an aqueous stream for the direct recycling of the crystallization mother liquors is also sent to the quench-ammonolysis (stream 25).

At the inlet to the quench-ammonolysis apparatus, the mass ratios between the aqueous streams 31, 34, 25 and the molten melamine stream 4 are selected so as to have, at the outlet, in the aqueous ammonia solution comprising purified melamine (stream 35), NH₃ concentrations ranging from 10% to 17% by mass (preferably from 12% to 15% by mass) and melamine concentrations ranging from 5% to 19% by mass (preferably from 7% to 15% by mass, and more preferably from 9 to 11% by mass)..

In the quench-ammonolysis apparatus the residence time is sufficient for almost completely eliminating the polycondensates present in stream 4, transforming them mostly into melamine..

The stream 7 exiting from QAL is sent to the filtration section F, composed of finishing filters that also complete the ammonolysis treatment. The stream exiting from section F (stream 8) is sent to the crystallization section Cr, where the melamine is precipitated by lowering the temperature to a value of approximately 40-50°C, obtaining an aqueous suspension of high-purity melamine (stream 9).

The low CO₂ content in the QAL inlet stream obtained through the section StrN allows the pH to be kept high during crystallization and therefore to operate under more unfavourable conditions for the precipitation of OATs. These favourable conditions for the crystallization of pure melamine are obtained: in the section StrN, by stripping the CO₂, using only NH₃ coming from the urea plant (stream 13, figure 1) and is sent back to it with the off-gases (stream 19 of figure 1). Furthermore, in the section StrN only the CO₂ is removed.

Stream 9 exiting from the section Cr is subjected to a solid/liquid separation in the section SLS, where the melamine crystals (stream 10) are separated from the crystallization mother liquors (stream 23) and sent to the drying and packaging sections (not shown in figure 1).

As already indicated, thanks to the shorter residence time of the melamine in the hot aqueous phase, the mother liquor stream 23 contains a limited amount of OATs; and it can therefore be partially recycled directly in the quench-ammonolysis section QAL without any treatment, in such a quantity as to remain under the saturation of the OATs in the crystallization.

The stream 23 exiting from the separator SLS is therefore divided into two streams (streams 24 and 25): stream 25 is recycled directly to the quench-ammonolysis section QAL, whereas stream 24 is sent to the deammoniation section AR.

The lower the quantity of OATs in the mother liquor, the higher the fraction of mother liquor that can be recycled directly to the section QAL will be, and the greater the savings on steam consumption and investment in the section AR and the direct recovery of melamine in the section QAL. According to the state of the art, the flow-rate of mother liquor directly recyclable to the section QAL (stream 25) is higher than 10% of the total flow-rate of mother liquor (stream 23).

The fraction of mother liquor not recycled directly (stream 24) is subjected to deammoniation in the section AR, which separates three streams: a stream of NH₃ substantially free of CO₂, to be recycled to the ammonolysis section QAL (stream 26); a stream rich in CO₂ (stream 27); an aqueous stream comprising melamine, OATs and substantially free of CO₂ and NH₃ (stream 28 of deammoniated mother liquor). The deammoniation AR can operate with any known method for the separation of mixtures of water-NH₃-CO₂.

The NH₃ stream 26 is extracted from the section AR preferably in the gaseous state and then mixed with a part of the aqueous stream 31 (shown in figure 1); in this way the condensation enthalpy of the NH₃ heats the resulting stream, with a positive effect on the heat balance of the section QAL and therefore on the steam consumption of the whole plant.

The stream rich in CO₂ 27 can be sent directly to the urea plant (figure 1), or recycled to a point in the melamine plant; it is preferably recycled to a point where the CO₂ contained therein cannot lower the pH in crystallization, and can ultimately leave the melamine plant, carrying with it less water than the above-mentioned direct sending to the urea plant (for example, after treatment to reduce its water content).

The stream of deammoniated mother liquor 28 is sent to a section OE for the elimination of OATs and to obtain an aqueous solution to be recycled to the sections for quench-ammonolysis QAL (stream 31) and off-gas washing OGQ (stream 32).

The section OE can function in several different ways:
a) in one case, the OATs are crystallized by cooling and neutralization with CO₂, and then separated from the stream 28 by ultrafiltration. This separation provides:
   - an aqueous stream 30 substantially free of OATs and rich in melamine, to be recycled to the sections QAL and OGQ (streams 31 and 32) thus also recovering the melamine contained therein;
   - a stream rich in suspended OATs, to be sent for decomposition to recover the organic compounds in the form of NH₃ and CO₂, obtaining a stream of water (stream 29) substantially free of impurities, which can be discharged or re-used at a suitable point in the system, for example in the place of demineralized water;
b) in the other case, the whole stream 28 is sent for decomposition to recover the organic compounds in the form of NH₃ and CO₂, obtaining a stream of water (stream 29) substantially free of impurities, which can be discharged or re-used at a suitable point in the system, and a stream of water 30 to be recycled to the sections QAL and, if present, also to the section OGQ (streams 31 and 32).

Starting from the state of the art previously described, i.e. from a low energy-consumption process for the production of high-purity melamine through the pyrolysis of urea, the objective of the present invention is to optimize the dissolution and ammonolysis step.

The present invention therefore relates to a process for the synthesis of high-purity melamine through the pyrolysis of urea, comprising a dissolution and ammonolysis step, wherein:
a) a stream 2 exiting from a reaction section R comprising reactor and post-reactor, at a temperature within the range of 360 to 400°C, preferably equal to 380°C, is fed to a dissolution step in aqueous ammonia solution, wherein the quantity of ammonia in the aqueous ammonia solution varies within the range of 15 to 28% by weight with respect to the total weight of the aqueous ammonia solution, in a dissolver MD at a temperature within the range of 160 to 180°C, preferably within the range of 170 to 172°C and at a pressure higher than 25 barg,
b) the solution 3 exiting from the dissolution and ammonolysis step a), comprises a quantity of dissolved melamine that varies within the range of 5 to 25% by weight, a quantity of polycondensates lower than 100 ppmw, which is fed to the subsequent filtration (F), crystallization (CR) and separation (SLS) steps.

The dissolution and ammonolysis step a) is preferably carried out at a pressure within the range of 25 to 40 barg.

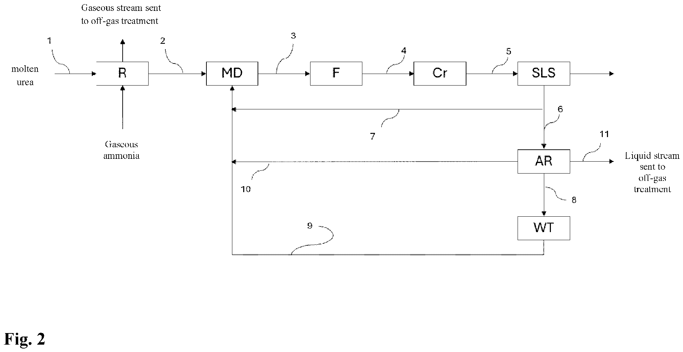

The process according to the present invention is represented in the block diagram of Figure 2.

Both the stream 2 fed to step a) and the solution 3 exiting from step a) are substantially free of CO₂ due to the passage of the stream (2) in the post-reactor in the section R.

By increasing the pressure from 25 barg to 40 barg, more preferably within the range of 30 to 35 barg, the ammonolysis of the polycondensates is promoted (cleavage of the polycondensate bond with the consequent introduction of the NH₃ molecule to reform the melamine). A higher pressure therefore allows a solution to be obtained at the end of step a) with a low polycondensate content, with a shorter residence time, under the same temperature conditions and ammonia concentration with the state-of-the-art processes. The reversible reaction of the polycondensates is the following:

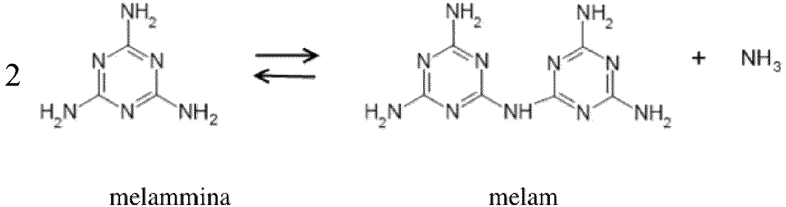

Step a) is preferably carried out under vigorous stirring to ensure complete mixing and not generate "dead spots" in the dissolution system.

Step a) is effected within a time of 20 to 30 minutes.

The concentration of NH₃ in the solution 3 exiting from step b) is preferably within the range of 13 to 26% by weight with respect to the total weight of solution 3 exiting from step b).

The quantity of OATs present in solution 3 exiting from step b) is less than 2000 ppmw, thanks to the reduction in the volumes and therefore residence times, in this section of the dissolution-ammonolysis step.

In step a), mother liquors recycled directly from the solid/liquid separation step (SLS) can be fed in a quantity greater than 15% by weight compared to the total flow-rate of mother liquors exiting from the solid-liquid separation system, preferably greater than 25% by weight.

Thanks to the reduction in the concentration of OATs, due to the reduction in the residence times, it was in fact possible to increase the percentage of mother liquors that are recycled directly to the dissolution and ammonolysis step.

Stream 2 leaving the post-reactor R is almost completely free of CO₂ and is sent to a specific apparatus for the dissolution of the raw melamine in water and for ammonolysis, i.e. for the destruction of the polycondensates, an apparatus defined as Melamine Dissolver - MD. The raw melamine enters the bottom of the apparatus MD, kept under vigorous stirring by a mechanical stirrer or a circulation pump, and is put in close contact with an ammonia solution (H₂O + NH₃), containing ammonia in a quantity within the range of 15 to 28% by weight with respect to the total weight of the aqueous ammonia solution, so as to guarantee a concentration of NH₃ exiting from the Melamine Dissolver MD within the range of 13 to 26% by weight.

The stirring and close contact between the streams can be obtained by means of distributors, static mixers, fillings, internal agitators, external circulation pumps or any other system typically used in the chemical industry for promoting the complete mixing of multiple fluid streams.

In step a) of the process according to the present invention, the raw melamine, free of CO₂, is totally dissolved at a temperature of 160-180°C, more preferably 170-172°C, generating a solution 3 at the outlet of the Melamine Dissolver MD which comprises a quantity of dissolved melamine that varies within the range of 5 to 25% by weight, free of CO₂, and a quantity of polycondensates lower than or equal to 100 ppmw.

The main advantage of the process according to the present invention, which comprises an optimized dissolution and ammonolysis step, is that of being capable of significantly reducing the residence times of melamine in aqueous solution, thus reducing the hydrolysis of the melamine itself and the consequent production of OATs (oxyaminotriazines). The reduction in the residence times is linked to the reduction in the volumes of the dissolution and ammonolysis apparatus, made possible by the particular operating conditions in which said dissolution and ammonolysis step is carried out according to the present invention.

A further advantage of the process according to the present invention which includes an optimized dissolution and ammonolysis step is that of obtaining a melamine solution almost completely free of polycondensates, with a significantly reduced quantity of OATs, less than 2000 ppmw, and with a consequent increase in the quality of the melamine produced.

The process according to the present invention is characterized by an optimized dissolution and ammonolysis step in which the pressure is higher than 25 barg and preferably ranges from 25 barg to 40 barg. This pressure range higher than 25 barg has increased the kinetics of the reaction

Polycondensates + NH₃ → Melamine

and it has been surprisingly found that this positive effect is no longer evident at pressures above 40 barg.

Having defined a maximum pressure limit for the dissolution and ammonolysis step, the process and relative equipment have therefore been optimized.

Having accelerated the kinetics of the dissolution and ammonolysis step, the relative Melamine Dissolver apparatus MD, as well as the filtration systems downstream of this, will also have significantly reduced dimensions linked to the requirement of lower residence times.

The process according to the present invention is therefore also characterized by an advantage of "economic savings" linked to the selection of equipment with a reduced volume and having defined a maximum pressure of 40 barg which avoids the use of equipment with higher project pressures which would require greater thicknesses. The process according to the present invention is therefore also characterized by an economic advantage in the form of a melamine having an equal or higher quality with respect to that obtained with the processes according to the state of the art.

The ammoniacal water stream fed to step a) of the process according to the present invention for the dissolution of melamine and the ammonolysis of the polycondensates comprises a quantity of ammonia within the range of 15 to 28% by weight, sufficient for obtaining a pH greater than 11 and thus avoiding the precipitation of OATs.

The optimization of the dissolution and ammonolysis step in terms of operating pressure also makes it possible to operate with higher concentrations of ammonia without risking the release of ammonia in the gas phase, keeping the ammonia in liquid phase and thus obtaining "free" ammonia in solution that reacts with the polycondensates to give melamine. Therefore, in accordance with the reversible reaction indicated above, by increasing the concentration of NH₃, the reaction shifts towards the reconversion of the polycondensates into melamine.

Furthermore, the reduction in the formation of OATs in the dissolution and ammonolysis section, due to the shorter residence time, allows a greater quantity of mother liquor coming from the separation step (again in a quantity which is such as to remain below the saturation of the OATs in an aqueous ammonia solution) to be recycled, obtaining a consequent significant energy saving as a lower quantity of ammonia water to be treated must be sent to the ammonia recovery treatment and to the water treatment (AR and WT in figure 2).

In fact, the lower the quantity of OATs in the mother liquor, the higher the fraction of mother liquor that can be recycled directly to the raw melamine dissolution step will be.

With the process according to the present invention, the flow-rate of mother liquors recycled directly to the dissolution step can be increased and exceed 15%, preferably greater than 25% by weight compared to the total flow-rate of mother liquors exiting from the solid-liquid separation system.

The fraction of mother liquors not recycled, on the other hand, is subjected to a deammoniation process that separates the pure ammonia, which is then recovered and recycled to generate ammoniacal water.

The conditions of the dissolution and ammonolysis step of the process according to the present invention allow a concentration of polycondensates lower than 100 ppmw to be reached even before the filter treatment (in which the ammonolysis action is completed); in the current state of the art a concentration lower than 100 ppmw of polycondensates could be reached only after the filtration section, i.e. only once the stream exiting from the ammonolysis section had passed through the carbon filter, completing the conversion of the polycondensates into melamine and, in any case, requiring higher volumes for longer residence times, while at the same time favouring the formation of OATs.

The stream 3 exiting from the section MD is sent to a filtration section F, consisting of filters suitable for removing undissolved impurities and active carbon reactors (carbon catalysts) with the aim of completing the ammonolysis treatment (Polycondensates +NH₃ → melamine).

The stream 4 exiting from F is then sent to a crystallization section Cr where the melamine is precipitated by lowering the temperature to a value of 40-50°C, obtaining a slurry of melamine having a very high purity (stream 5) suspended in a solution of water and ammonia (mother liquors).

The almost total absence of CO₂ exiting from the post-reactor (the CO₂ was stripped with NH₃) allows the pH to be kept high (above 11) even in the crystallization phase and therefore operating under conditions much more unfavorable for the precipitation of OATs.

The stream 5, exiting from the crystallization step Cr, is subjected to a solid/liquid separation step SLS, wherein the melamine crystals are separated from the mother liquor, and then sent for drying and packaging.

The fraction of mother liquor not recycled directly (stream 6) is subjected to deammoniation in the section AR, which separates three streams: a stream of NH₃ substantially free of CO₂, to be recycled to the section MD (stream 10) to generate ammoniacal water for dissolution and ammonolysis; a stream containing water, ammonia and CO₂ (stream 11); an aqueous stream comprising melamine, OATs and substantially free of CO₂ and NH₃ (stream 8). The deammoniation AR can operate with any known method for the separation of water-NH₃-CO₂ mixtures.

The stream 10 of NH₃ is extracted from the section AR preferably in the gaseous state and then mixed with a part of the aqueous stream, stream 9, obtained from the water treatment (WT) to generate the ammoniacal dissolution water. Stream 11, on the other hand, is a stream in the liquid state that can be sent directly to the urea plant, or recycled to a point in the melamine plant; it is preferably recycled to a point where the CO₂ contained therein cannot lower the pH in crystallization, and can ultimately leave the melamine plant, taking with it less water than the above-mentioned direct sending to the urea plant (for example, after treatment that reduces its water content).

The stream of water free of CO₂ and NH₃ (stream 8) is sent to a water treatment section (WT) to eliminate the OATs and obtain an aqueous solution to be recycled to the dissolution section (MD) (stream 9).

The optimized dissolution and ammonolysis step of the process according to the present invention can be applied both to a process such as that described in EP2385043B1 (Figure 1) and to melamine synthesis processes that comprise a washing treatment of the off-gases with molten urea.

The process can also be applied in plants constructed according to the state of the art, such as those necessary for effecting the processes schematically represented in figure 1, by applying modifications that are not too complex (so-called "revamping").

The following embodiment example (Example 2) is provided for illustrating the present invention and does not limit the protection scope defined by the enclosed claims.

### EXAMPLE 1 (comparative)

The stream of liquid melamine exiting from the stripper/post reactor (7.8 t/h) at a temperature of 380°C is sent to the quench-ammonolyzer for purification in aqueous solution with NH₃, under the following operating conditions: temperature 172°C, pressure 25 barg and concentration of NH₃ 15 % by mass. 94 t/h of an aqueous solution at 8% by mass of melamine exit from the quench-ammonolyzer-

This solution, leaving the quench-ammonolysis section, is sent to the filters from which a solution with less than 100 ppm by mass of polycondensates exits; this latter solution is then sent to the crystallizer, which operates at a temperature of 45°C, a pressure of 0.5 barg, CO₂ concentration of about 0.15% by mass and a pH of about 11.5. The suspension leaving the crystallizer is sent to a solid/liquid separator (centrifuge), which separates the crystallization mother liquors from the melamine.

In a 7.5 t/h melamine production plant, the volume required for dissolution is approximately 83 m³ (Quench + Ammonolyzer), and therefore the melamine solution treated in this system will have a residence time of approximately 48 minutes. The ammonia concentration exiting from the section QAL is 13.5% by mass and the pressure is always maintained at 25 barg (not higher). Under these conditions, the stream exiting from the QAL system has the following concentration of polycondensates and OATs:
Polycondensates 100 ppmw;
OATs 3100 ppmw.

The concentration of OATs of this stream directed towards the filtration/crystallization/separation system is therefore equal to 3100 ppmw.

### EXAMPLE 2

By increasing the pressure to 30 barg, in order to always produce 7.5 t/h of melamine, the total volume of the dissolver-ammonolyzer system was reduced to 50 m³. The melamine solution therefore has a residence time of 30 minutes in the dissolver..

In particular, the liquid melamine stream exiting from the stripper/post-reactor (7.8 t/h) at a temperature of 380°C is sent to the dissolver for dissolution and ammonolysis MD in aqueous solution with NH₃, under the following operating conditions: temperature 172°C, pressure 30 barg and NH₃ concentration 15% by mass.

The ammonia concentration exiting from the section MD is equal to 13.5% and the pressure is equal to 30 barg. Under these conditions, the stream exiting from the dissolver MD, before passing through the filtration section, has the following concentration of polycondensates and OATs:
Polycondensates less than 100 ppmw;
OATs 2000 ppmw.

This stream is sent directly to the filtration/crystallization/separation system, without further treatment as the higher pressure has guaranteed a concentration of polycondensates of less than 100 ppmw and a concentration of OATs equal to 2000 ppmw. Having reduced the residence time to 60% thanks to the reduction in the volumes, the concentration of OATs of this stream directed towards the filtration/crystallization/separation system is in fact lower (2000 ppmw) than that of Example 1 (3100 ppmw).

### EXAMPLE 3 (comparative)

Repeating Example 2 by only changing the pressure that has been set at 45 barg, the stream leaving the dissolver MD has the following concentration of polycondensates and OATs:
Polycondensates 100 ppmw;
OATs 2000 ppmw.

This stream is sent directly to the filtration/crystallization/separation system without further treatment. Despite having increased the operating pressure compared to Example 2, it is evident that the concentration of polycondensates has not substantially changed under all other conditions (maintaining the same volumes and ammonia concentration as Example 2). This increase in pressure obviously entails disadvantages in terms of the complexity of the equipment, increased thicknesses of the equipment, etc. which make the use of pressures higher than 40 barg not optimal.

Therefore, comparing the example according to the present invention (Example 2) with the example according to the state of the art (Example 1), it can be noted that the volume of the dissolution and ammonolysis section has been reduced to 60%, thanks to the increase in pressure that has favoured the ammonolysis reaction of the polycondensates with the formation of melamine. The reduction to 60% of the volume also involves a significant reduction in the hydrolysis of melamine to OATs and allows the production of a melamine with a higher qualitative value.

## Claims

1. A process for the synthesis of high-purity melamine by the pyrolysis of urea, comprising a dissolution and ammonolysis step, wherein:
a) a stream (2) exiting from a reaction section (R) comprising a reactor and post-reactor, at a temperature ranging from 360 to 400°C, preferably equal to 380°C, is fed to a dissolution step in aqueous ammonia solution, wherein the quantity of ammonia in the aqueous ammonia solution varies within the range of 15 to 28% by weight with respect to the total weight of the aqueous ammonia solution, in a dissolver (MD) at a temperature within the range of 160 to 180°C and at a pressure higher than 25 barg,
b) the solution (3) exiting from the dissolution and ammonolysis step a), comprises a quantity of dissolved melamine that varies within the range of 5 to 25% by weight, a quantity of polycondensates lower than 100 ppmw, which is fed to the subsequent filtration (F), crystallization (CR) and separation (SLS) steps.

2. The process according to claim 1, wherein the dissolution and ammonolysis step a) is carried out at a temperature within the range of 170 to 172°C.

3. The process according to claim 1, wherein the dissolution and ammonolysis step a) is carried out at a pressure within the range of 25 to 40 barg.

4. The process according to claim 1, wherein the dissolution and ammonolysis step a) is carried out at a pressure within the range of 30 to 35 barg.

5. The process according to any of the previous claims, wherein step a) is carried out under vigorous stirring.

6. The process according to any of the previous claims, wherein step a) is carried out for a time ranging from 20 to 30 minutes.

7. The process according to any of the previous claims, wherein the concentration of NH₃ in the solution (3) exiting from step b) is within the range of 13 to 26% by weight with respect to the total weight of the solution (3) exiting from step b).

8. The process according to any of the previous claims, wherein the quantity of OATs present in the solution (3) exiting from step b) is less than or equal to 2000 ppmw.

9. The process according to any of the previous claims, wherein, in the dissolution step a), mother liquors recycled directly from the solid/liquid separation step (SLS) are fed in a quantity greater than 15%, preferably greater than 25% by weight, with respect to the total flow-rate of mother liquors exiting from the solid-liquid separation system.

10. The process according to any of the previous claims, wherein the stream (2) fed to step a) and the solution (3) exiting from step a) are free of CO₂.

## Amended claims

### Amended claims in accordance with Rule 137(2) EPC.

1. A process for the synthesis of high-purity melamine by the pyrolysis of urea, comprising a dissolution and ammonolysis step, wherein:
a) a stream (2) exiting from a reaction section (R) comprising a reactor and post-reactor, at a temperature ranging from 360 to 400°C, preferably equal to 380°C, is fed to a dissolution step in aqueous ammonia solution, wherein the quantity of ammonia in the aqueous ammonia solution varies within the range of 15 to 28% by weight with respect to the total weight of the aqueous ammonia solution, in a dissolver (MD) at a temperature within the range of 160 to 180°C and at a pressure higher than 25 barg,
b) the solution (3) exiting from the dissolution and ammonolysis step a), comprises a quantity of dissolved melamine that varies within the range of 5 to 25% by weight, a quantity of polycondensates lower than 100 ppmw and the quantity of OATs is less than or equal to 2000 ppmw, which is fed to the subsequent filtration (F), crystallization (CR) and separation (SLS) steps.

2. The process according to claim 1, wherein the dissolution and ammonolysis step a) is carried out at a temperature within the range of 170 to 172°C.

3. The process according to claim 1, wherein the dissolution and ammonolysis step a) is carried out at a pressure within the range of 25 to 40 barg.

4. The process according to claim 1, wherein the dissolution and ammonolysis step a) is carried out at a pressure within the range of 30 to 35 barg.

5. The process according to any of the previous claims, wherein step a) is carried out under vigorous stirring.

6. The process according to any of the previous claims, wherein step a) is carried out for a time ranging from 20 to 30 minutes.

7. The process according to any of the previous claims, wherein the concentration of NH₃ in the solution (3) exiting from step b) is within the range of 13 to 26% by weight with respect to the total weight of the solution (3) exiting from step b).

8. The process according to any of the previous claims, wherein, in the dissolution step a), mother liquors recycled directly from the solid/liquid separation step (SLS) are fed in a quantity greater than 15%, preferably greater than 25% by weight, with respect to the total flow-rate of mother liquors exiting from the solid-liquid separation system.

9. The process according to any of the previous claims, wherein the stream (2) fed to step a) and the solution (3) exiting from step a) are free of CO₂.
